(19) Europäisches Patentamt

European Patent Office

Office européen des brevets

(11) **EP 1 773 893 B1**

(12) **EUROPEAN PATENT SPECIFICATION**

(45) Date of publication and mention
of the grant of the patent:
**02.06.2010 Bulletin 2010/22**

(51) Int Cl.:
**C08F 2/22** *(2006.01)*     **C08F 220/02** *(2006.01)*
**C08F 220/10** *(2006.01)*     **A61F 9/00** *(2006.01)*
**G02C 7/04** *(2006.01)*

(21) Application number: **04749259.0**

(22) Date of filing: **03.08.2004**

(86) International application number:
**PCT/SG2004/000237**

(87) International publication number:
**WO 2006/014138 (09.02.2006 Gazette 2006/06)**

(54) **POLYMER HAVING INTERCONNECTED PORES FOR DRUG DELIVERY AND METHOD**

POLYMER MIT MITEINANDER VERBUNDENEN POREN ZUR ARZNEISTOFFZUFUHR UND
VERFAHREN

POLYMÈRE AYANT DES PORES INTERCONNECTÉS POUR ADMINISTRATION DE MÉDICAMENT
ET PROCÉDÉ

(84) Designated Contracting States:
**AT BE BG CH CY CZ DE DK EE ES FI FR GB GR
HU IE IT LI LU MC NL PL PT RO SE SI SK TR**

(43) Date of publication of application:
**18.04.2007 Bulletin 2007/16**

(73) Proprietor: **Agency for Science, Technology and
Research
Singapore 138632 (SG)**

(72) Inventors:
• **CHOW, Pei Yong Edwin
Singapore 138669 (SG)**
• **YANG, Yi Yan
Singapore 138669 (SG)**

(74) Representative: **Baldock, Sharon Claire
Boult Wade Tennant
Verulam Gardens
70 Gray's Inn Road
London WC1X 8BT (GB)**

(56) References cited:
**EP-A- 0 449 450     EP-A2- 0 882 996
WO-A-97/35904     WO-A-2005/007717
WO-A1-97/35904     US-B1- 6 451 348
US-B1- 6 652 581**

• **LIU J ET AL: "SYNTHESIS AND
POLYMERIZATION OF A NONIONIC
SURFACTANT: POLY(ETHYLENE OXIDE)
MACROMONOMER" JOURNAL OF NON-
CRYSTALLINE SOLIDS, NORTH-HOLLAND
PUBLISHING COMPANY, AMSTERDAM, NL, vol.
A33, no. 3, 1996, pages 337-352, XP008051357
ISSN: 0022-3093**

**Description**

**FIELD OF THE INVENTION**

The present invention relates to drug delivery, polymers for drug delivery, and related methods.

**BACKGROUND OF THE INVENTION**

Currently, most eye medications or ophthalmic drugs are applied directly to the eye in the form of drops. However, there are drawbacks associated with using eye drops to deliver medications. Typically, about 95% of the medication applied is lost after application. Eye drops applied topically mix with tears, which then drain into the nasal cavity and, from there, enter into the bloodstream and other organs, where the drugs can cause side effects. Additionally, the release rate of the drug declines rapidly following an initial high rate, giving rise to inconsistent dosage.

Contact lenses have been used as a vehicle for delivering ophthalmic drugs. One of the conventional approaches is to soak the lenses in the drug solution and then insert the lenses into the eyes of a patient. The contact lenses may be solid or may have a cavity for receiving the drug solution. This approach produces unsatisfactory results because the drug release rate drops quickly over time.

Another approach that has been described is to encapsulate an ophthalmic drug in nanoparticles dispersed in the contact lenses. When the contact lens is put on the eye, the drug diffuses into and migrates through the contact lens and into the post-lens tear film. However, this approach has certain drawbacks. The manufacturing processes for this kind of contact lenses involves a multi-step encapsulation procedure and can be complex and expensive. The amount of drug that can be encapsulated is small. The encapsulated drug may affect the transparency of the resulting contact lens and it is also difficult to control the release rate.

**SUMMARY OF THE INVENTION**

A transparent and porous polymer having interconnected pores is provided for drug delivery. The pores may be filled with a liquid such as water. The drug is dispersed in the polymer and is releasable when the polymer is in contact with a liquid. Due to the interconnection of the pores, the drug may be released at a relatively steady rate. The rate of drug release is also dependent on the particular porous structure of the polymer, which can be specifically formed to achieve a desired rate of release.

Therefore, an aspect of the invention provides a method of forming a polymer comprising polymerizing a bicontinuous microemulsion comprising water, a monomer, and a surfactant copolymerizable with said monomer, to form a porous polymer comprising a polymer matrix defining interconnected pores filled by said water, wherein said microemulsion further comprises a drug such that, when said porous polymer is formed, said drug is dispersed in one or both of said polymer matrix and said pores and is releasable therefrom when said porous polymer is in contact with a liquid. The drug can be an ophthalmic drug. The pores may have a pore diameter of about 10 to about 100 nm.

In another aspect of the invention, there is provided a polymer formed in accordance with the method described in the preceding paragraph.

In another aspect of the invention, there is provided a polymer comprising a polymer matrix defining interconnected pores distributed throughout the polymer; and a drug dispersed in one or both of the polymer matrix and the pores, the drug being releasable therefrom when the polymer is in contact with a liquid. The pores may have a pore diameter of about 10 to about 100 nm and may be filled with water. The drug may be an ophthalmic drug.

In another aspect of the invention, there is provided a drug delivery device comprising a transparent and porous polymer defining interconnected pores; and an ophthalmic drug dispersed in one or both of the polymer and the pores, wherein the ophthalmic drug is releasable from the device when the device is in contact with a liquid. The drug delivery device may be a contact lens or an artificial cornea. The pores may have a pore diameter of about 10 to about 100 nm and may be filled with water.

In another aspect of the invention, there is provided a method of delivering an ophthalmic drug, comprising loading the ophthalmic drug in an ophthalmic device comprising a transparent and porous polymer, the polymer defining interconnected pores, the ophthalmic drug dispersed in one or both of the polymer and the pores, wherein the ophthalmic drug is releasable from the device when the device is in contact with a liquid. The ophthalmic device is a contact lens or an artificial cornea.

Other aspects and features of the present invention will become apparent to those of ordinary skill in the art upon review of the following description of specific embodiments of the invention in conjunction with the accompanying figures.

## BRIEF DESCRIPTION OF THE DRAWINGS

In the figures, which illustrate exemplary embodiments of the invention,

**FIG. 1** is a schematic diagram illustrating a contact lens made of a exemplary polymer of the invention;

**FIG. 2** shows a scanning electron microscopic image of an exemplary polymer of the invention;

**FIG. 3** is a schematic diagram illustrating the structure of a bicontinuous microemulsion;

**FIG. 4** is a graph showing the results of percentage weight content measurements of exemplary polymers;

**FIGS. 5** and **6** are line graphs showing the drug release rates of exemplary polymers; and

**FIGS. 7A** and **7B** show images of human fibroblast cells grown on exemplary polymers.

## DETAILED DESCRIPTION OF EXEMPLARY EMBODIMENTS

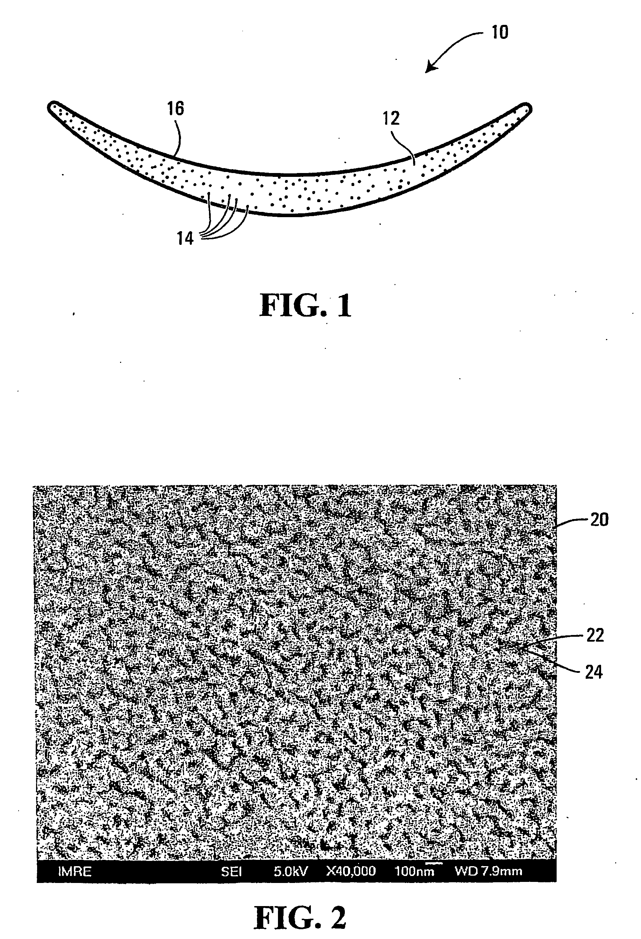

Referring to **Fig. 1,** an exemplary embodiment of the invention is a contact lens **10** made of a transparent and porous polymer **12** having an ophthalmic drug **14** incorporated therein. When the contact lens **10** is put on the eye, a surface **16** of the contact lens **10** is in contact with the post-lens tear film and the ophthalmic drug **14** is released from the contact lens **10** into the post-lens tear film at a desirable rate.

As used herein, the term "transparent" broadly describes the degree of transparency that is acceptable for a contact lens or like devices, for example the degree of transmission of visible light through the polymer equivalent to that of other materials employed in the manufacture of contact lenses or other ophthalmic devices.

As illustrated in **Fig. 2,** which is a Scanning Electron Microscopic image of the internal structure of an exemplary polymer suitable for use as the polymer **12,** the polymer has a polymer matrix **20** (shown as the bright portions) defining interconnected elongate pores **22** (shown as the dark portions). Pores are interconnected when at least some of them are joined or linked with each other to form one or more continuous networks. The pores **22** can be filled with a fluid **24** such as water or air.

The pores shown in **Fig. 2** have a pore diameter of about 30 to 80 nm. The pores 22 may have round or other cross-sectional shapes and may have different sizes. As used herein, a pore diameter refers to the average or effective diameter of the cross-sections of the pores. The effective diameter of a cross-section that is not circular equals the diameter of a circular cross-section that has the same cross-sectional area as that of the non-circular cross-section. In some embodiments, such as when the polymer is swellable when the pores are filled with water, the sizes of the pores may change depending on the water content in the polymer. When the polymer is dried, some or all of the pores may be filled or partially filled by a gas such as air. The polymer **12** may thus behave like a sponge. In alternative embodiments, the pore diameter may be in the range from about 10 to 100 nm when the polymer **12** is in a dry condition wherein the water content of the polymer **12** is at or near minimum.

The pores **22** may be randomly distributed. Some of the pores **22** may be closed pores, meaning that they are not connected or joined with other pores or open to the surfaces of the polymer. It is not necessary that all of the pores **22** are interconnected since as more fully discussed below, depending on use, polymers can be prepared to have more or less interconnected pores as would be understood by a skilled person

The ophthalmic drug **14** (not identifiable in the image of **Fig. 2**) is dispersed in the polymer matrix **20** or in the pores **22** which is filled for example with a liquid such as water, or in both. The ophthalmic drug **14** is releasable from the polymer **12** when it is in contact with a liquid such as a post-lens tear film. The ophthalmic drug **14** may diffuse through the liquid in the interconnected pores **22** from an inner region to a surface region of the polymer **12,** such as the surface **16** of the contact lens **10.** If the ophthalmic drug **14** is dispersed in the polymer matrix **20,** the ophthalmic drug **14** may also enter the liquid in the pores **22** after migrating or diffusing through the polymer matrix **20.**

The release of the ophthalmic drug **14** is facilitated by the interconnected pores **22** and the liquid in the pores. The drug release rate can be controlled in part by altering the size and the degree of interconnection of the pores **22** and the properties of the liquid in the pores **22.** Thus, the polymer **12** can be conveniently used to deliver an ophthalmic drug in a controlled manner.

The ophthalmic drug **14** is capable of travelling or migrating within the polymer **12** such as by diffusion. In general, the drug molecules or particles move in random directions but when there is a concentration gradient, there is a net flow of the drug molecules or particles from the high concentration region to the low concentration region. The drug molecules or particles may travel faster in a liquid filling the pores **22** than in the polymer matrix **20.**

Thus, returning to **Fig. 1,** the ophthalmic drug **14** is releasable from the contact lens **10** when the contact lens **10** is in contact with a liquid such as a post-lens tear film when the contact lens **10** is put on the eye. As can be understood, the drug molecules or particles at the surface **16** of the contact lens **10** may enter into the post-lens tear film, creating a concentration gradient within the contact lens **10,** lower near the surface **16** and higher away from the surface **16.** Thus, some drug molecules initially located away from the surface **16** will gradually move to the surface **16** and enter the post-lens tear film.

As can be understood, the drug release rate is dependent on the structure and properties of the polymer **12.**

The release rate may be increased when there are more pores, or when there are more interconnected pores. While a larger pore diameter may provide faster release initially, a smaller pore diameter with increased length of interconnected pores can provide a more steady release at a similar rate. The release rate may also be affected by the manner in which the drug is incorporated in the polymer **12**. When the drug is initially located in the polymer matrix **20**, the rate may be slower. When the drug is initially located in the fluid **24,** the rate may be higher. Thus, the drug delivery rate may be controlled by controlling the pore structure and the manner in which the drug is incorporated in the polymer **10**.

The polymer **10** may be prepared by polymerizing a bicontinuous microemulsion of one or more copolymerizable monomers, one or more surfactants copolymerizable with at least one of the monomers, and water, such that the resulting polymer has interconnected pores filled with water. An ophthalmic drug may be dispersed in the microemulsion and the microemulsion may also include a polymerization initiator or a cross-linker, or both.

As is understood in the art, "microemulsion" refers to a thermodynamically stable dispersion of one liquid phase into another liquid phase. The microemulsion may be stabilized by an interfacial film of surfactant. One of the two liquid phases is hydrophilic or lipophobic (such as water) and the other is hydrophobic or lipophilic (such as oil). Typically, the droplet or domain diameters in microemulsions are about 100 nanometers or less, and thus the microemulsions are transparent. A microemulsion can be continuous or bicontinuous. The preparation of microemulsions is known in the art. For example, a mixture of the components may be dispersed to form a microemulsion by standard techniques such as sonication, vortexing, or other agitation techniques for creating microdroplets of the different phases within the mixture. Alternatively, the mixture may be passed through a filter having pores on the nanometer scale so as to create fine droplets.

Depending on the proportions of various components and the hydrophile-lipophile value of the surfactant, the droplets can be swollen with oil and dispersed in water (referred to as normal or O/W microemulsion), or swollen with water but dispersed in oil (referred to as inverse or W/O microemulsion), or the microemulsion can be bicontinuous.

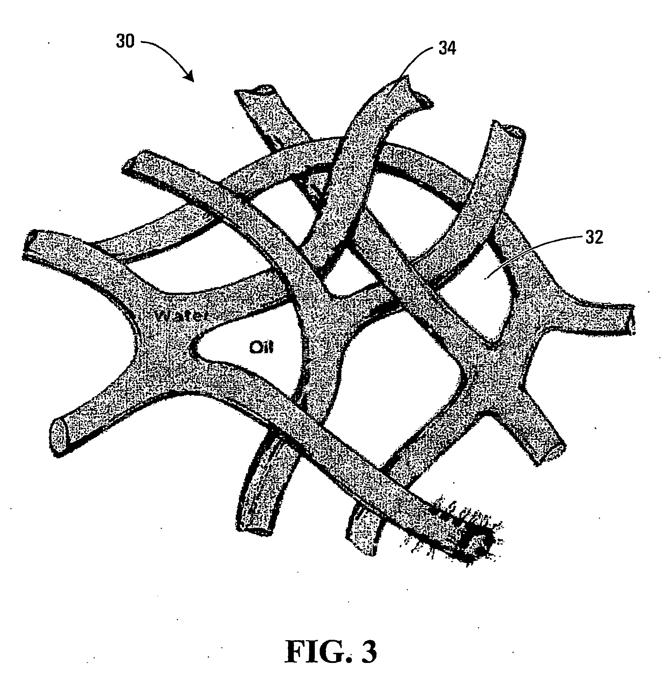

A bicontinous microemulsion is formed to prepare the polymer **20**. An exemplary structure of a bicontinuous microemulsion **30** is illustrated in **Fig. 3,** wherein oil domains **32** (containing the monomers) and aqueous domains **34** (containing water) are randomly distributed and respectively interconnected, extending in all three dimensions. When the oil domains **32** are polymerized, the presence of the aqueous domains **34** results in interconnected pores filled with the water that was present in the aqueous domains **34.**

The monomers for forming biocontinous microemulsion can be any suitable monomer known to persons skilled in the art, which is capable of copolymerizing with another monomer to form a copolymer. While the monomer is copolymerizable with another monomer such as the surfactant, the monomer may also be polymerizable with itself. The type and amount of the monomer that may be employed to prepare a suitable bicontinous microemulsion will be known to a skilled person. Exemplary monomers are ethylenically unsaturated monomers including methyl methacrylate (MMA), 2-hydroxylethyl methacrylate (HEMA), 2-hydroxylethyl acrylate, monocarboxylic acids such as acrylic acid (AA) and methacrylic acid (MA), glycidyl methacrylate (GMA), and silicone-type monomers. Suitable combinations of these monomers can also be used.

A polymerizable surfactant is capable of polymerizing with itself or with other monomeric compounds to form a polymer. The surfactant for the mixture can be any suitable surfactant that can co-polymerize with at least one of the monomers in the microemulsion. As can be appreciated, when the surfactant is copolymerized into the polymer, there is no need to separate the surfactant from the polymer after polymerization. This can be advantageous as the polymer formation process is simplified. The surfactant can be anionic, non-ionic or zwitterionic. Exemplary surfactants include poly(ethylene oxide)-macromonomer (PEO- macromonomer), such as $\omega$-methoxy poly(ethylene oxide)$_{40}$ undecyl $\alpha$-methacrylate macromonomer denoted herein as $C_1$-PEO-$C_{11}$-MA-40. The chain length of the macromonomer can be varied. For example, the macromonomer may be in the form of $CH_3O(CH_2CH_2O)_x$-$(CH_2)_nV$, or may be zwitterionic surfactants such as $SO_3(CH_2)_m{}^+NCHCHCHN(CH_2)_nV$, where m is an integer ranging from 1 to 20, n is an integer ranging from 6 to 20, x is an integer ranging from 10 to 110, and V is (methyl)acrylate or another copolymerisable unsaturated group.

The choice and weight ratio of the particular monomer and surfactant for a given application may depend on the application. Generally, they should be chosen such that the resulting polymer is suitable and compatible with the environment in which the polymer is to be used and has the desired properties.

The water in the microemulsion can be pure water or a water-based liquid. The water may optionally contain various additives having specific properties. Such additives can be selected for achieving one or more desired properties in the resulting polymer, and can include one or more of a drug, a protein, an enzyme, a filler, a dye, an inorganic electrolyte, and a pH adjuster.

As will be understood by a skilled person in the art, a nanoporous and transparent polymer matrix can be obtained when the components of the microemulsion are in appropriate ratios and the droplets or domains have appropriate sizes. As is known to persons skilled in the art, to determine the appropriate proportions of the components suitable for forming a bicontinuous microemulsion, a ternary phase diagram for the monomer, water and the surfactant may be prepared. The region on the diagram corresponding to single-phase microemulsion may be identified and the proportions of the components may be so chosen such that they fall within the identified region. A person skilled in the art will be able to adjust the proportions according to the diagram in order to achieve a certain desirable property in the resulting polymer. Further, the formation of a bicontinous microemulsion can be confirmed using techniques known to persons skilled in the art. For example, the conductivity of the mixture may increase substantially when the microemulsion is bicontinuous. The conductivity of the mixture may be measured using a conductivity meter after titrating a 0.1 M sodium chloride solution into the mixture.

Suitable bicontinuous microemulsions can be formed when proportions of the components are respectively from about 15 to about 50 % for water, from about 5% to about 40% for the monomer, and from about 10% to about 50% for the surfactant, all percentages by weight (denoted wt% hereafer). Persons skilled in the art will understand how to combine different monomers and surfactants in different ratios to achieve the desired effect on the various properties of the resulting polymer, for example to improve the mechanical strength or hydrophilicity of the resulting polymer.

For medical applications, the polymer should be safe and biocompatible with human cells. For use as contact lenses, it is desirable that the polymer is permeable to fluids such as gases (e.g. $O_2$ and $CO_2$), various salts, nutrients, water and diverse other components of the tear fluid. The presence of nanopores distributed throughout the polymer facilitates the transport.of gases, molecules, nutrients and minerals through the eye and the surroundings. It will be appreciated that the polymers according to the invention can provide a more controlled delivery of drugs, thereby improving the performance of therapeutic contact lenses formed from the polymer.

Therefore, in different embodiments, a drug such as an ophthalmic drug can be incorporated into the microemulsion. The drug may be dispersed in the aqueous domains or in the oil domains of the microemulsion, or in both domains including at the interface of the two domains. When the drug is initially dispersed in the oil domains, it is likely dispersed in the polymer matrix after polymerization. When the drug is initially dispersed in the aqueous domains, it is likely dispersed in the water in the pores after polymerization. Drugs that can be incorporated in the polymer can vary and can be either hydrophilic or hydrophobic, water soluble or water insoluble. A person skilled in the art will understand how different drugs will be dispersed in the microemulsion depending on their properties such as hydrophilicity or lipophilicity.

Exemplary ophthalmic drugs include anti-glaucoma agents such as a beta adrenergic receptor antagonist, e.g. timolol maleate, and other therapeutic agents such as antibiotic agents, antibacterial agents, anti-inflammatory agents, anaesthetic agents, anti-allergic agents, polypeptides and protein groups, lubricating agents, and any combination or mixture of the above.

The amount of the drug to be included can be determined based on various factors. In general, the drug should have a concentration suitable for providing the desired therapeutic dosage, as would be known in the art. For ophthalmic drug delivery, the transparency and clarity of the resulting polymer material is one of the factors. A high drug loading may affect the phase equilibrium of the microemulsion precursor and the resulting polymer material may not be sufficiently transparent. Tests show that transparent polymers can be prepared when up to about 0.3 wt% drug is loaded. Another factor is the rate of release. Experiments show that higher loading resulted in higher release rate. A further factor is the mechanical properties of the resulting polymer. Experiments show that concentration of the drug affects the polymer's mechanical properties.

The microemulsion may be polymerized to form a transparent and porous polymer wherein the ophthalmic drug is incorporated, either in the polymer or the pores, or both.

The microemulsion may be polymerized by standard techniques known to a skilled person. For example, it may be polymerized by heat, the addition of a catalyst, by irradiation of the microemulsion or by introduction of free radicals into the microemulsion. The method of polymerization chosen will be dependent on the nature of the components of the microemulsion.

Polymerization of the microemulsion may involve the use of a catalyst. The catalyst may be any catalyst or polymerization initiator that promotes polymerization of the monomers and the surfactant. The specific catalyst chosen may depend on the particular monomers, and polymerizable surfactant used or the method of polymerization. For example, polymerization can be achieved by subjecting the microemulsion to ultraviolet (UV) radiation if a photo-initiator is used as a catalyst. Exemplary photoinitiators include 2,2-dimethoxy-2-phenyl acetophenone (DMPA) and dibenzylketone. A redox-initiator may also be used. Exemplary redox-initiators include ammonium persulphate and N,N,N',N'-tetramethylethylene diamine (TMEDA). A combination of photo-initiator and redox-initiator may also be used. In this regard, including in the mixture an initiator can be advantageous. The polymerization initiator may be about 0.1 wt% to about 0.4 wt% of the microemulsion.

To promote cross-linking between polymer molecules in the resulting polymer, a cross-linker may be added to the mixture. Suitable cross-linkers include ethylene glycol dimethacrylate (EGDMA), diethylene glycol dimethacrylate and diethylene glycol diacrylate. As can be understood, the more the polymer molecules are cross-linked, the more difficult it is for the drug to diffuse or migrate through the polymer, thereby resulting in a slower release of the drug.

The microemulsion may be formed into a desired end shape and size prior to polymerization. For example, a sheet material may be formed by pouring or spreading the mixture into a layer of a desired thickness or by placing the mixture between glass plates prior to polymerization. The mixture may also be formed into a desired shape such as a rod, for example, by pouring the mixture into a mold or cast prior to polymerizing.

After polymerization, the polymer may be rinsed and equilibrated with water to remove un-reacted monomers and the drug that has not been incorporated into the polymer. A small percentage of the drug incorporated in the polymer may be lost during rinsing but the amount lost can be limited by controlling the duration of rinsing. Further, the initial concentration can be adjusted accordingly so that the final concentration provides the desired rate of release. The rinsed polymer can be optionally dried and sterilized in preparation for use in a medical or clinical application. Both drying and sterilization can be accomplished in any suitable manner, which is known to person of skill in the art. In some embodiments, both drying and sterilization can be effected at a low temperature so as not to adversely affect the drug, for example using ethyleneoxide gas or UV radiation.

The ophthalmic drug can be released steadily from the polymer when the polymer is in contact with a liquid. The release rate of the drug can be controlled by selecting the appropriate monomers and their proportional amounts.

Since only one polymerization step is required to prepare the polymer incorporating a drug, the process can be simple and inexpensive.

The resulting polymer can be used to form contact lenses or other ophthalmic devises or articles such as artificial corneas. The contact lenses formed can be used for vision correction or eye colour modification, or can be diabetic contact lenses. The artificial corneas can be used for corneal wound healing applications.

Therefore, a method of delivering an ophthalmic drug may include loading an ophthalmic drug in an ophthalmic device made of a transparent and porous polymer defining interconnected pores. The pores may be filled with a fluid such as water. The ophthalmic drug is dispersed in one or both of the polymer and the pores. The ophthalmic drug is releasable from the device when the device is in contact with a liquid such as a post-lens tear film. The device can be a contact lens and the ophthalmic drug is releasable from the contact lens when the contact lens is placed on the eye. The device can also be an artificial cornea.

Conveniently, the polymer according to various embodiments of the invention can be made compatible with human dermal fibroblasts cells and mechanically strong and can be advantageously used to manufacture contact lenses for placement on the eye.

The polymer can have various desirable physical, chemical, and biochemical properties. To illustrate, the properties of exemplary polymers are described below. These samples were formed as follows.

For each sample, a precursor mixture was first prepared. The principle components of the mixtures are listed in Table 1. The weight percentages listed were calculated based on the total weights of the listed components only.

**Table 1 Contents of Microemulsion Components (wt%)**

| Sample | $C_1$-PEO-$C_{11}$-MA-40 | MMA | HEMA | Water |
|---|---|---|---|---|
| Polymer-20-T10 | 40.0 | 20.0 | 20.0 | 20.0 |
| Polymer-30-T10 | 35.0 | 17.5 | 17.5 | 30.0 |
| Polymer-40-T10 | 30.0 | 15.0 | 15.0 | 40.0 |
| Polymer-20-T20 | 40.0 | 20.0 | 20.0 | 20.0 |
| Polymer-20-T30 | 40.0 | 20.0 | 20.0 | 20.0 |
| Polymer-20 | 40.0 | 20.0 | 20.0 | 20.0 |
| Polymer-30 | 35.0 | 17.5 | 17.5 | 30.0 |
| Polymer-40 | 30.0 | 15.0 | 15.0 | 40.0 |

These components were mixed by vortex-mixing, respectively for each sample.

Further, various amounts of timolol maleate were added. For samples ending in "T10", "T20" or "T30", 10, 20 or 30 mg oftimolol maleate were respectively added to each gram of the corresponding mixture. In addition, 0.3 wt% DMPA was added as UV-initiator and 0.5 to 1 wt% EGDMA was added as cross-linker, both percentages based on the total weight of the polymerizable groups.

The single-phase region of a microemulsion of $C_1$-PEO-$C_{11}$-MA-40, HEMA, MMA, and water was determined by titrating water to various compositions of the microemulsion, in a screw-capped test tube. Each sample was vortex-mixed and allowed to equilibrate in a temperature-controlled environment at 23°C. The clear-turbid points were used to establish the phase boundary of the microemulsion region in a phase-diagram. A rough demarcation of the bicontinuous region was further deduced from conductivity measurements using a conductivity meter after titrating a 0.1M sodium chloride solution into the mixture. Tests showed that a microemulsion can be formed when the aqueous content of the mixture is in the range of about 20 wt% to 60 wt% and that the conductivity of the mixture increased rapidly when the aqueous content increased from below about 20 wt% to above about 20 wt%. It is believed that the sharp increase in conductivity at about 20 wt% was due to the formation of numerous interconnected conducting channels in the microemulsion, characteristic of a bicontinuous microemulsion.

The microemulsion precursors were pre-purged with nitrogen gas to ensure there was no significant oxygen present, which, as is known, may inhibit polymerization. The precursor for a sample was placed between two glass plates or in a polymethacrylate mold. The plates or mold with the precursor was then placed in a Rayonet™ photoreactor chamber and was subjected to UV-radiation (254 nm) at about 35°C to effect polymerization for about two hours.

After polymerization, the liquid microemulsion transformed into a solid polymer having interconnected pores filled with the water initially present in the microemulsion. No undesirable side products were observed after polymerization.

The polymerized sample material, after being removed from the plates or mold, was washed to remove unpolymerized residue monomer, surfactant, timolol maleate, and etc. The sample material was washed successively with deionized distilled water at temperatures between the room temperature to about 60°C for one to two hours. At the end of washing, no substantial amount of un-reacted monomers and timolol was present in the sample material, as confirmed by the absence of UV absorption bands between 190 and 350 nm in the washing solution.

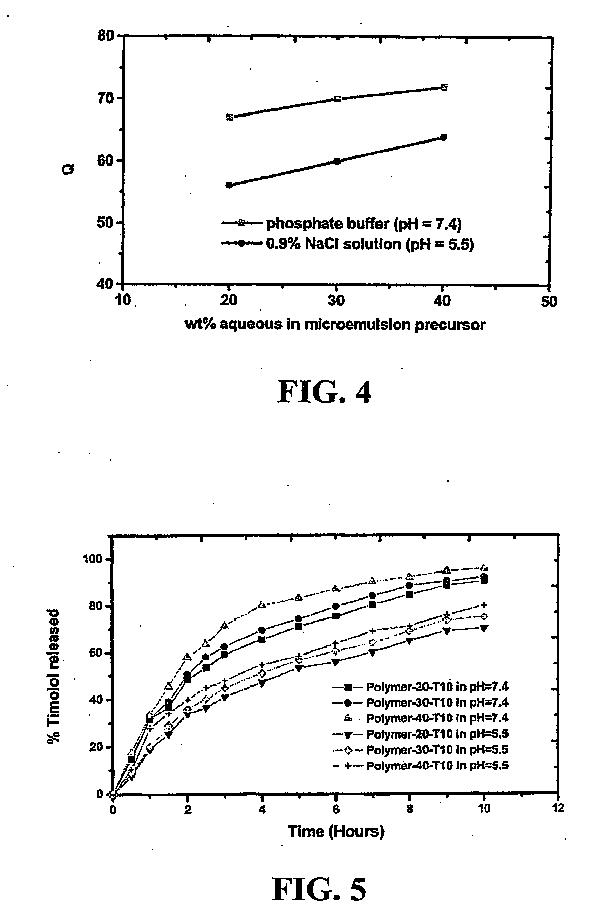

Measurements show that the un-loaded samples (Polymer-20, Polymer-30, and Polymer-40) had high percentage water contents (Q) after reaching equilibrium in a liquid medium. The results of swelling measurements carried out at 37°C in phosphate buffer solutions and lacrimal fluid (0.9% NaCl) are shown in **Fig. 4.** Q was calculated as follows:

$$Q = (W_S - W_d) \times 100 / W_S, \qquad (1)$$

where $W_s$ is the swollen weight and $W_d$ is the dry weight of the sample. As is apparent from **Fig. 4,** the higher the initial water content in the precursor mixture, or the higher the pH value in the liquid medium, the higher the Q. Without being limited to a particularly theory, The observed high swelling ability is likely due to the complete dissociation of the functional groups of the monomers at higher pH (-7), which increases the electrostatic repulsion between the negatively charged ions, thus expanding the interconnected porous network. The high swelling is also likely due to high porosity in the sample material, as it has been observed that polymer systems with high water content have high porosity.

The glass transition temperatures (Tg) of the sample materials were determined by scanning calorimeter characterization. 5-10 mg of each sample was sealed in an aluminum pan. The sealed sample was heated from room temperature to about 200°C, cooled to about 0°C, and then heated again up to about 200°C, at a rate of 10°C/min under nitrogen. The results are summarized in Table 2.

**Table 2 Glass Transition Temperature**

| Sample | Tg (°C) |
|---|---|
| Polymer-20-T10 | 104 |
| Polymer-30-T10 | 102 |
| Polymer-40-T10 | 100 |
| Polymer-20-T20 | 108 |
| Polymer-20-T30 | 112 |
| Polymer-20 | 90 |
| Polymer-30 | 86 |
| Polymer-40 | 84 |

As can be seen, the glass transition temperatures increased when the samples were loaded with a drug. This is likely due to the filler reinforcement effect. The filler (drug) retards the chain mobility of the polymer segment, thus increasing Tg. Further, the lower the polymer content, the lower the Tg (e.g. comparing Polymer-40-T10 with Polymer-20-T10).

The dynamo-mechanical properties of the sample materials were evaluated at 25°C and 80°C. The temperature dependence of the elastic (G') and viscous moduli (G") of the sample materials were recorded from an angular frequency of 1 rad/s by measuring these parameters while increasing the temperature from 25°C to 80°C at 1°C/min. The results are listed in Table 3.

**Table 3 Mechanical Characterization**

| Sample | G' (Mpa) | | G" (Mpa) | |
|---|---|---|---|---|
| | 25°C | 80°C | 25°C | 80°C |
| Polymer-20-T10 | 0.290 | 0.137 | 0.124 | 0.086 |
| Polymer-30-T10 | 0.115 | 0.069 | 0.071 | 0.016 |
| Polymer-40-T10 | 0.092 | 0.031 | 0.054 | 0.007 |
| Polymer-20 | 0.091 | 0.049 | 0.030 | 0.011 |
| Polymer-30 | 0.077 | 0.055 | 0.019 | 0.007 |
| Polymer-40 | 0.039 | 0.026 | 0.007 | 0.004 |

At temperatures well below the Tg, the sample materials had high values of G' and G", which decreased at higher temperatures near the Tg. As can be appreciated by person skilled in the art, these G' and G" values were suitable for contact lenses because a proper balance of comfort and visual performance can be achieved.

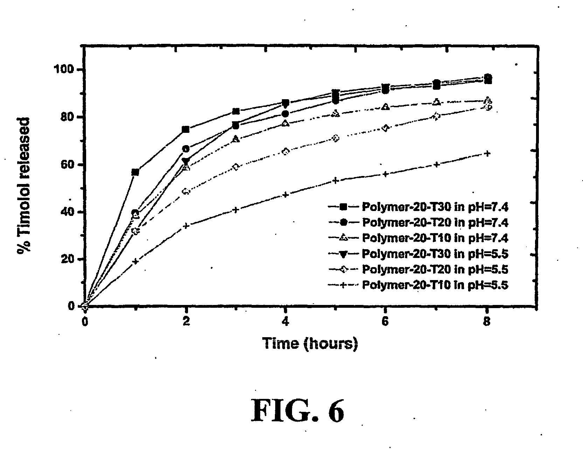

The release rate of timolol from the loaded sample materials were measured at 37°C in a phosphate buffer (pH 7.4) solution and a 0.9% NaCl solution (pH 5.5) respectively. The solutions were sampled at regular intervals and the sample solutions were measured spectrophotometrically. The results are shown in **Figs. 5** and **6.** As can be seen, the release rate is relatively steady over many hours. The rates depended on the solution medium, the type and amount of polymer present in the materials, and the loading of the drug. The release rate of timolol maleate was faster when a larger amount of the drug was loaded. Again, without being limited to a particularly theory, the higher release rate is likely due to the greater swelling ability of the polymer material and its high porosity.

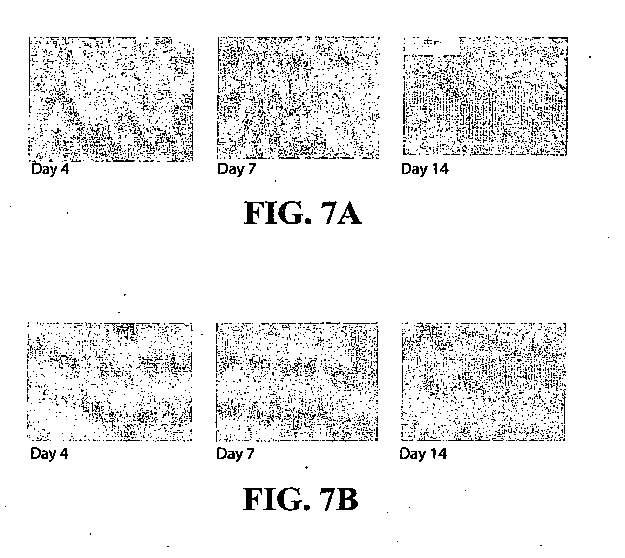

To test the biocompatibility of the sample materials, Human Fibroblast Cells were seeded on the sample materials for a period of 14 days and cell proliferation and differentiation were monitored. The cells were initially round in shape when seeded. After a few days the cells were elongate in shape, as can be seen from the images shown in **Figs. 7A** (on Polymer-20) and **7B** (on Polymer-30), indicating that the cells adhered and grew well on the sample material substrates, which in turn indicates that the sample materials are compatible with the cells and cell growth.

Other features, benefits and advantages of the present invention not expressly mentioned above can be understood from this description and the drawings by those skilled in the art.

Although only exemplary embodiments of this invention have been described above, those skilled in the art will readily appreciate that many modifications are possible therein without materially departing from the novel teachings and advantages of this invention. For example, a drug other than an ophthalmic drug can be incorporated in the polymer. Other medicinal or therapeutic drugs can be incorporated in a polymer formed in accordance with the invention for controlled release. The polymer may be formed into a corresponding desired shape and size suitable for delivery of such other drugs. Further, a drug may be loaded after the polymer is formed. For example, the pores of the polymer may be filled or purged with a liquid containing a desired drug after the polymer is formed. In some applications, such as when a slow release rate is desirable or where necessitated by the natures of various components of the polymer, the drug may be encapsulated in particles before being dispersed in the polymer, wherein the drug can migrate or diffuse through the walls of the particles and thus be released from the polymer. Nanoencapsulation of drugs are known, for example as described in U.S. Patent Application Publication 2004-0096477 (May 20, 2004)

The invention, rather, is intended to encompass all such modification within its scope, as defined by the claims.

**Claims**

**1.** A method of forming a polymer, comprising:

polymerizing a bicontinuous microemulsion comprising water, a monomer, and a surfactant copolymerizable with said monomer, to form a porous polymer comprising a polymer matrix defining interconnected pores filled by said water, wherein said microemulsion further comprises a drug such that, when said porous polymer is formed, said drug is dispersed in at least said polymer matrix and said drug in said polymer matrix is releasable from said polymer matrix into said pores when said porous polymer is in contact with a liquid.

**2.** The method of claim 1, wherein said drug is an ophthalmic drug.

3. The method of claim 1 or claim 2, wherein said pores have a pore diameter of 10 to 100 nm.

4. The method of any one of claims 1 to 3, wherein the proportion of said water is from 15% to 50% by weight, the proportion of said monomer is from 5% to 40% by weight, and the proportion of said surfactant is from 10% to 50% by weight, based on total weight of said bicontinuous microemulsion.

5. The method of any one of claims 1 to 4, wherein said microemulsion further comprises a cross-linker.

6. The method of claim 5 wherein the cross-linker is ethylene glycol dimethacrylate (EGDMA).

7. The method of any one of claims 1 to 6, wherein said microemulsion further comprises a polymerization initiator.

8. The method of claim 7, wherein said polymerization initiator is a photo-initiator.

9. The method of claim 8 wherein the photo-initiator is 2,2-dimethoxy-2-phenyl acetophenone (DMPA).

10. The method of claim 9, wherein said polymerizing comprises subjecting said microemulsion to ultraviolet radiation.

11. The method of any one of claims 1 to 10, wherein said monomer is ethylenically unsaturated.

12. The method of claim 11, wherein said monomer is methyl methacrylate (MMA), 2-hydroxyethyl methacrylate (HEMA), or a combination of MMA and HEMA.

13. The method of any one of claims 1 to 12, wherein said surfactant is a non-ionic surfactant.

14. The method of any one of claims 1 to 13, wherein said surfactant is a poly(ethylene oxide)-macromonomer.

15. The method of claim 14 wherein the surfactant is $\omega$-methoxy poly(ethylene oxide)$_{40}$ undecyl $\alpha$-methacrylate macromonomer ($C_1$-PEO-$C_{11}$-MA-40).

16. A polymer comprising:

   a polymer matrix defining interconnected pores distributed throughout said polymer; and
   a drug dispersed in at least said polymer matrix, said drug being releasable therefrom when said polymer is in contact with a liquid.

17. The polymer of claim 16, wherein said drug is an ophthalmic drug.

18. The polymer of claim 17, wherein said pores have a pore diameter of 10 to 100 nm.

19. A drug delivery device comprising:

   a transparent and porous polymer defining interconnected pores; and
   an ophthalmic drug dispersed in said polymer,

   wherein said ophthalmic drug is releasable from said drug delivery device when said drug delivery device is in contact with a liquid.

20. The drug delivery device of claim 19, which is a contact lens or an artificial cornea.

21. The drug delivery device of claim 19 or claim 20, wherein said pores have a pore diameter of 10 to 100 nm.

22. Use of an ophthalmic device comprising a transparent porous polymer, said polymer defining interconnecting pores in a method of delivering an ophthalmic drug which method comprises loading said ophthalmic drug in said ophthalmic device to produce a drug delivery device in accordance with claim 19.

23. The use of claim 22, wherein said ophthalmic device is a contact lens or an artificial cornea.

**Patentansprüche**

1. Verfahren zur Bildung eines Polymers, umfassend:

   das Polymerisieren einer bikontinuierlichen Mikroemulsion, die Wasser, ein Monomer und ein Tensid, das mit dem Monomer copolymerisierbar ist, umfasst, um ein poröses Polymer zu bilden, das eine Polymermatrix umfasst, die miteinander verbundene Poren, die mit dem Wasser gefüllt sind, definiert, wobei die Mikroemulsion weiterhin ein Arzneimittel umfasst, so dass, wenn das poröse Polymer gebildet wird, das Arzneimittel mindestens in der Polymermatrix dispergiert wird, und das Arzneimittel in der Polymermatrix aus der Polymermatrix in die Poren freigesetzt werden kann, wenn das poröse Polymer in Kontakt mit einer Flüssigkeit ist.

2. Verfahren nach Anspruch 1, wobei das Arzneimittel ein ophthalmisches Arzneimittel ist.

3. Verfahren nach Anspruch 1 oder Anspruch 2, wobei die Poren einen Porendurchmesser von 10 bis 100 nm aufweisen.

4. Verfahren nach einem der Ansprüche 1 bis 3, wobei der Anteil des Wassers von 15 Gewichts-% bis 50 Gewichts-% ist, der Anteil des Monomers von 5 Gewichts-% bis 40 Gewichts-% ist und der Anteil des Tensids von 10 Gewichts-% bis 50 Gewichts-% ist, bezogen auf das Gesamtgewicht der bikontinuierlichen Mikroemulsion.

5. Verfahren nach einem der Ansprüche 1 bis 4, wobei die Mikroemulsion weiterhin ein Vernetzungsmittel umfasst.

6. Verfahren nach Anspruch 5, wobei das Vernetzungsmittel Ethylenglykoldimethacrylat (EGDMA) ist.

7. Verfahren nach einem der Ansprüche 1 bis 6, wobei die Mikroemulsion weiterhin einen Polymerisationsinitiator umfasst.

8. Verfahren nach Anspruch 7, wobei der Polymerisationsinitiator ein Photoinitiator ist.

9. Verfahren nach Anspruch 8, wobei der Photoinitiator 2,2-Dimethoxy-2-phenyl-acetophenon (DMPA) ist.

10. Verfahren nach Anspruch 9, wobei das Polymerisieren das Aussetzen der Mikroemulsion gegenüber ultravioletter Strahlung umfasst.

11. Verfahren nach einem der Ansprüche 1 bis 10, wobei das Monomer ethylenisch ungesättigt ist.

12. Verfahren nach Anspruch 11, wobei das Monomer Methylmethacrylat (MMA), 2-Hydroxyethylmethacrylat (HEMA) oder eine Kombination aus MMA und HEMA ist.

13. Verfahren nach einem der Ansprüche 1 bis 12, wobei das Tensid ein nichtionisches Tensid ist.

14. Verfahren nach einem der Ansprüche 1 bis 13, wobei das Tensid ein Poly(ethylenoxid)-Makromonomer ist.

15. Verfahren nach Anspruch 14, wobei das Tensid w-Methoxy-poly(ethylenoxid)$_{40}$-undecyl-$\alpha$-methacrylat-Makromonomer (C$_1$-PEO-C$_{11}$-MA-40) ist.

16. Polymer, umfassend:

    eine Polymermatrix, die miteinander verbundene Poren definiert, die über das ganze Polymer verteilt sind; und
    ein Arzneimittel, das mindestens in der Polymermatrix dispergiert ist, wobei das Arzneimittel daraus freigesetzt werden kann, wenn das Polymer in Kontakt mit einer Flüssigkeit ist.

17. Polymer nach Anspruch 16, wobei das Arzneimittel ein ophthalmisches Arzneimittel ist.

18. Polymer nach Anspruch 17, wobei die Poren einen Porendurchmesser von 10 bis 100 nm aufweisen.

19. Vorrichtung zur Arzneimittelzufuhr, umfassend:

ein transparentes und poröses Polymer, das miteinander verbundene Poren definiert; und

ein ophthalmisches Arzneimittel, das in dem Polymer dispergiert ist, wobei das ophthalmische Arzneimittel aus der Vorrichtung zur Arzneimittelzufuhr freigesetzt werden kann, wenn die Vorrichtung zur Arzneimittelzufuhr in Kontakt mit einer Flüssigkeit ist.

20. Vorrichtung zur Arzneimittelzufuhr nach Anspruch 19, die eine Kontaktlinse oder eine künstliche Hornhaut ist.

21. Vorrichtung zur Arzneimittelzufuhr nach Anspruch 19 oder Anspruch 20, wobei die Poren einen Porendurchmesser von 10 bis 100 nm aufweisen.

22. Verwendung einer ophthalmischen Vorrichtung, die ein transparentes poröses Polymer umfasst, wobei das Polymer miteinander verbundene Poren definiert, in einem Verfahren zur Zufuhr eines ophthalmischen Arzneimittels, wobei das Verfahren das Beladen der ophthalmischen Vorrichtung mit dem ophthalmischen Arzneimittel umfasst, um eine Vorrichtung zur Arzneimittelzufuhr gemäß Anspruch 19 herzustellen.

23. Verwendung nach Anspruch 22, wobei die ophthalmische Vorrichtung eine Kontaktlinse oder eine künstliche Hornhaut ist.

**Revendications**

1. Procédé de formation d'un polymère, comprenant :

la polymérisation d'une microémulsion bicontinue comprenant de l'eau, un monomère, et un agent tensio-actif copolymérisable avec ledit monomère, afin de former un polymère poreux comprenant une matrice polymère définissant des pores interconnectés remplis par ladite eau, dans lequel ladite microémulsion comprend en outre un médicament de sorte que, lorsque ledit polymère poreux est formé, ledit médicament est dispersé dans au moins ladite matrice polymère et ledit médicament dans ladite matrice polymère peut être libéré de ladite matrice polymère dans lesdits pores lorsque ledit polymère poreux est en contact avec un liquide.

2. Procédé de la revendication 1, dans lequel ledit médicament est un médicament ophtalmique.

3. Procédé de la revendication 1 ou de la revendication 2, dans lequel lesdits pores ont un diamètre de pores de 10 à 100 nm.

4. Procédé de l'une quelconque des revendications 1 à 3, dans lequel la proportion de ladite eau va de 15% à 50% en poids, la proportion dudit monomère va de 5% à 40% en poids, et la proportion dudit agent tensio-actif va de 10% à 50% en poids, sur la base d'un poids total de ladite microémulsion bicontinue.

5. Procédé de l'une quelconque des revendications 1 à 4, dans lequel ladite microémulsion comprend en outre un agent de réticulation.

6. Procédé de la revendication 5, dans lequel l'agent de réticulation est du diméthacrylate d'éthylène glycol (EGDMA).

7. Procédé de l'une quelconque des revendications 1 à 6, dans lequel ladite microémulsion comprend en outre un initiateur de polymérisation.

8. Procédé de la revendication 7, dans lequel ledit initiateur de polymérisation est un photo-initiateur.

9. Procédé de la revendication 8 dans lequel le photo-initiateur est un 2,2-diméthoxy-2-phényl acétophénone (DMPA).

10. Procédé de la revendication 9, dans lequel ladite polymérisation comprend le fait de soumettre ladite microémulsion à un rayonnement ultraviolet.

11. Procédé de l'une quelconque des revendications 1 à 10, dans lequel ledit monomère est éthyléniquement insaturé.

12. Procédé de la revendication 11, dans lequel ledit monomère est un méthacrylate de méthyle (MMA), un (2-hydroxyéthyl)méthacrylate (HEMA), ou une combinaison de MMA et de HEMA.

**13.** Procédé de l'une quelconque des revendications 1 à 12, dans lequel ledit agent tensio-actif est un agent tensio-actif non ionique.

**14.** Procédé de l'une quelconque des revendications 1 à 13, dans lequel ledit agent tensio-actif est un macromonomère de poly(oxyde d'éthylène).

**15.** Procédé de la revendication 14 dans lequel l'agent tensio-actif est un macromonomère de $\omega$-méthoxy poly(oxyde d'éthylène) $_{40}$ undécyle $\alpha$-méthacrylate ($C_1$-PEO-$C_{11}$-MA-40) .

**16.** Polymère comprenant :

une matrice polymère définissant des pores interconnectés distribués à travers ledit polymère ; et
un médicament dispersé dans au moins ladite matrice polymère, ledit médicament pouvant être libéré de celle-ci, lorsque ledit polymère est en contact avec un liquide.

**17.** Polymère de la revendication 16, dans lequel ledit médicament est un médicament ophtalmique.

**18.** Polymère de la revendication 17, dans lequel lesdits pores ont un diamètre de pores de 10 à 100 nm.

**19.** Dispositif de distribution de médicament comprenant :

un polymère poreux et transparent définissant des pores interconnectés ; et
un médicament ophtalmique dispersé dans ledit polymère,

dans lequel ledit médicament ophtalmique peut être libéré dudit dispositif de distribution de médicament lorsque ledit dispositif de distribution de médicament est en contact avec un liquide.

**20.** Dispositif de distribution de médicament de la revendication 19, qui est une lentille de contact ou une cornée artificielle.

**21.** Dispositif de distribution de médicament de la revendication 19 ou de la revendication 20, dans lequel lesdits pores ont un diamètre de pores de 10 à 100 nm.

**22.** Utilisation d'un dispositif ophtalmique comprenant un polymère poreux transparent, ledit polymère définissant des pores s'interconnectant dans un procédé de distribution d'un médicament ophtalmique lequel procédé comprend le fait de remplir ledit médicament ophtalmique dans ledit dispositif ophtalmique afin de produire un dispositif de distribution de médicament selon la revendication 19.

**23.** Utilisation de la revendication 22, dans laquelle ledit dispositif ophtalmique est une lentille de contact ou une cornée artificielle.

10

16 12

14

**FIG. 1**

20

22

24

IMRE    SEI    5.0kV    X40,000    100nm    WD 7.9mm

**FIG. 2**

**FIG. 3**

**FIG. 4**

**FIG. 5**

**FIG. 6**

Day 4   Day 7   Day 14

## FIG. 7A

Day 4   Day 7   Day 14

## FIG. 7B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040096477 A